# EUROPEAN PATENT SPECIFICATION

(11) **EP 4 069 710 B1**
(45) Date of publication and mention of the grant of the patent: **04.10.2023**
(21) Application number: 20828523.9
(22) Date of filing: 03.12.2020
(51) Int. Cl.: C07F 15/00, A61P 35/00, A61K 31/555

(54) **PD(I) COMPLEXES, PROCESS FOR THEIR PREPARATION AND USE THEREOF AS ANTITUMOUR AGENTS**
PD(I)-KOMPLEXE, VERFAHREN ZU IHRER HERSTELLUNG UND IHRE VERWENDUNG ALS ANTITUMORMITTEL
COMPLEXES DE PD (I), LEUR PROCÉDÉ DE PRÉPARATION ET LEUR UTILISATION EN TANT QU'AGENTS ANTITUMORAUX

(30) Priority: 05.12.2019 IT 201900023079
(43) Date of publication of application: 12.10.2022
(73) Proprietor: Universita' Ca' Foscari Venezia, 30123 Venezia (VE) (IT)
(72) Inventor: VISENTIN, Fabiano, 35010 Trebaseleghe (IT); SCATTOLIN, Thomas, 30037 Scorzè (IT); BORTOLAMIOL, Enrica, 31020 Vidor (IT); RIZZOLIO, Flavio, 33081 Aviano (IT)
(74) Representative: Palladino, Saverio Massimo
(86) International application number: PCT/EP2020/084360
(87) International publication number: WO 2021/110790

(56) References cited:
- WO-A1-2019/158978
- BOYD PETER D. W. ET AL: "Reduction of a Chelating Bis(NHC) Palladium(II) Complex to [{[mu]-bis(NHC)}2Pd2H]+: A Terminal Hydride in a Binuclear Palladium(I) Species Formed under Catalytically Relevant Conditions", ANGEWANDTE CHEMIE INTERNATIONAL EDITION, vol. 49, no. 36, 23 August 2010 (2010-08-23), pages 6315-6318, XP055784259, ISSN: 1433-7851, DOI: 10.1002/anie.201003177
- RUIHU WANG ET AL: "Syntheses and Characterization of Unsymmetric Dicationic Salts Incorporating Imidazolium and Triazolium Functionalities", INORGANIC CHEMISTRY, vol. 45, no. 16, 1 August 2006 (2006-08-01), pages 6396-6403, XP055721746, EASTON, US ISSN: 0020-1669, DOI: 10.1021/ic060822i
- HECKENROTH M ET AL: "Synthesis and structural analysis of palladium biscarbene complexes derived from bisimidazolium ligand precursors", INORGANICA CHIMICA ACTA, ELSEVIER BV, NL, vol. 359, no. 6, 10 April 2006 (2006-04-10), pages 1929-1938, XP028068846, ISSN: 0020-1693, DOI: 10.1016/J.ICA.2005.10.045 [retrieved on 2006-04-10]
- HEMMERT CATHERINE ET AL: "Synthesis, structures, and antimalarial activities of some silver(I), gold(I) and gold(III) complexes involvingN-heterocyclic carbene ligands", EUROPEAN JOURNAL OF MEDICINAL CHEMISTRY, ELSEVIER, AMSTERDAM, NL, vol. 60, 7 December 2012 (2012-12-07), pages 64-75, XP028983520, ISSN: 0223-5234, DOI: 10.1016/J.EJMECH.2012.11.038

## Description

### FIELD OF THE INVENTION

The present invention relates to the sector of chemotherapy compounds and in particular of the metal complexes used for the purpose. The invention also relates to a process for the synthesis of these complexes and the therapeutic use thereof.

### STATE OF THE ART

The discovery of the antitumour properties of cisplatin (cis-diamine-dichloroplatinum(II)), and its approval by the *Food and Drug Administration* in 1978, paved the way for the use of inorganic compounds as chemotherapy agents. However, cisplatin has some important limitations, connected on one hand with its nephro- and neuro-toxicity and, on the other hand, with its inefficacy towards some types of tumours or induced resistance phenomena on others.

Platinum compounds have appeared on the market that have fewer side effects (carboplatin and oxaliplatin); however, these compounds, having the same action mechanism proposed for cisplatin (platination of DNA) turned out to be ineffective towards cisplatin-resistant tumours.

Many research groups have therefore focused in recent decades on the synthesis and study of the antitumour properties of compounds containing different metals from platinum. Among the most investigated compounds in this area, gold and ruthenium can be listed, whereas palladium compounds have only recently been taken into consideration.

Although it belongs to the same group as platinum, this element enables complexes to be obtained that are more soluble in water and moreover in some cases have a different primary target from the DNA. This latter characteristic explains the marked anti-proliferative activity of some of these compounds towards cisplatin-resistant cell lines.

A critical feature of palladium compounds relates to their higher hydrolysis speed with respect to platinum ones, with a consequent dissociation of the ligands and serious speciation problems (i.e. the formation of different species containing palladium derived from the starting one); this can cause greater collateral toxicity, due to the lack of control over the species effectively introduced into the organism.

However, it has recently been demonstrated that the use of ancillary ligands strongly anchored to a metal centre of Pd(II) significantly reduces or eliminates the problem connected with the dissociation of ligands in solution.

A category of ligands that have been widely studied in this sense in recent times is *N-*heterocyclic carbenes (NHCs). Pd(II) complexes containing these ligands have provoked a certain amount of interest, thanks to their high anti-proliferative activity on different, both cisplatin-sensitive and cisplatin-resistant, tumour lines. Pd(II) complexes of this type are for example the subject matter of patent applications US 2018/194789 A1 and CN 108558952 A.

There are not however, in the state of the art, any studies relating to the antitumour properties of palladium in oxidation state +1 (Pd(I)). These complexes are definitely less common than Pd(II) ones, since they are generally not very stable both in the solid state and in solution, being sensitive to oxygen and water. Among the few examples present in the literature, there are dimeric species of Pd(I), in which there is a Pd-Pd bond and in which every Pd atom is complexed by a phosphine and is bonded to a halogen atom. Examples of complexes of this type are reported in patent applications US 2012/190873 A1, US 2014/187803 A1 and WO 2018/073559 A1. These compounds have been studied so far only in view of their application as catalysts for chemical reactions, e.g. in cross-coupling processes.

Dinuclear Pd-complexes are also known from BOYD PETER D. W. ET AL in ANGEW. CHEMIE IEE 2010, 49(36), 6315-6318; RUIHU WANG ET AL in INORGANIC CHEMISTRY 2006, 45(16), 6396-6403; HECKENROTH M ET AL in INORGANICA CHIMICA ACTA 2006, 359(6), 1929-1938 and also from WO2019/158978. The last document (WO2019/158978) also discloses the use of such complexes in treating cancer. Similar silver and gold dinuclear complexes are known from HEMMERT CATHERINE ET AL in EUROPEAN JOURNAL OF MEDICINAL CHEMISTRY 2012, 60, 64-75.

### SUMMARY OF THE INVENTION

The object of the present invention is to provide a new family of palladium (I) complexes provided with antitumour activity, and to provide the process for the production thereof.

These objects are achieved with the present invention which in a first aspect thereof relates to a compound of general formula (A) or of general formula (B): 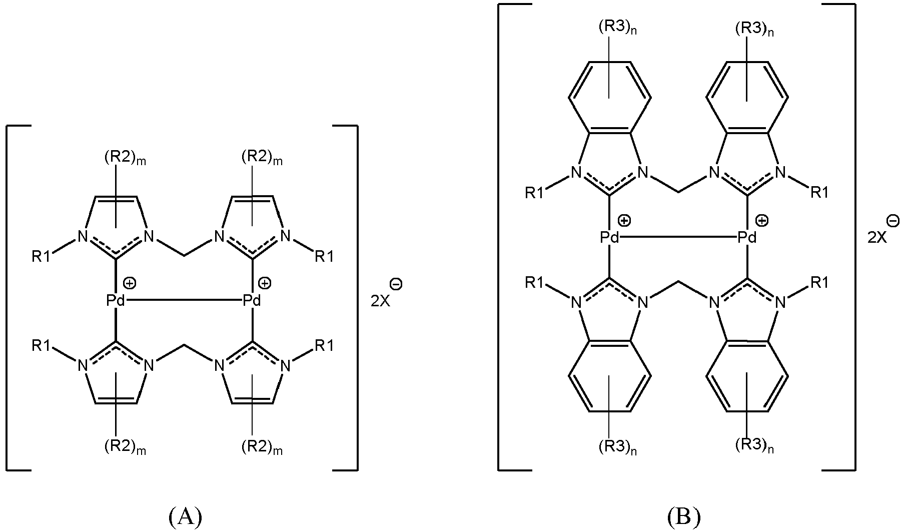 wherein:
- R1 is an alkyl radical or a phenyl radical optionally substituted with one or more C₁-C₃ alkyl groups;
- R2 is a linear or branched C₁-C₆ alkyl radical;
- R3 is a linear or branched C₁-C₆ alkyl radical;
- m = 0, 1 or 2;
- n = 0, 1, 2, 3 or 4;
- the notation 2X^{Θ} means two pharmaceutically acceptable monovalent anions or a pharmaceutically acceptable bivalent anion.

In a second aspect thereof, the invention relates to a process for producing compounds of general formula (A) or (B), which consists in reacting a compound of general formula (C) or of general formula (D) with a palladium (II) complex, according to the following diagrams: 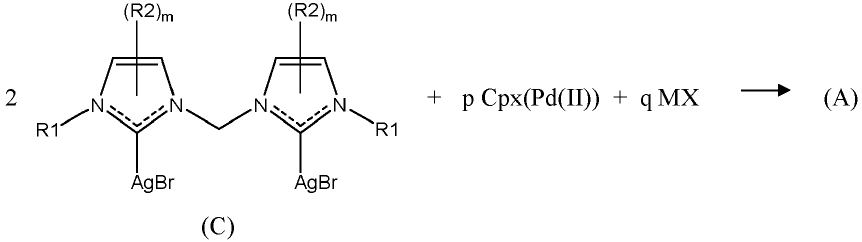 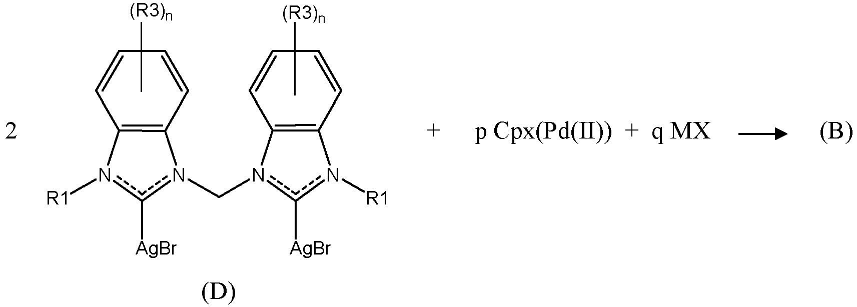 wherein R1, R2, R3, m, n and X have the meanings reported above, Cpx(Pd(II)) indicates a complex of palladium in oxidation state +2, and MX generally indicates a salt containing the monovalent or bivalent anion X defined above. The stoichiometric coefficient p is 2 if Cpx(Pd(II)) contains a single Pd atom, and is 1 if Cpx(Pd(II)) contains two Pd atoms, so as to keep a 1:1 molar ratio between compound (C) or (D) and Pd. The stoichiometric coefficient q is 1 in the event in which the anion X is bivalent and is 2 in the event in which the anion X is monovalent, regardless of the oxidation number of the metal M. The salt will have the formula M₁X₁ in the event in which the metal M and the anion X are both monovalent (respectively, cation with oxidation number +1 and anion with charge -1) or both bivalent (cation with oxidation number +2 and anion with charge -2); formula M₁X₂ (cation with oxidation number +2 and anion with charge -1); or formula M₂X₁ (cation with oxidation number +1 and anion with charge -2).

### BRIEF DESCRIPTION OF THE DRAWINGS

The invention will be illustrated below with reference to the Figures, in which:
- Figure 1 shows the ¹H-NMR spectrum of a compound of the invention;
- Figure 2 shows the ¹³C{¹H}-NMR spectrum of the same compound of Figure 1;
- Figure 3 shows a perspective image of the three-dimensional structure of the compound of Figure 1, reconstructed with the single-crystal X-ray diffraction method.

### DETAILED DESCRIPTION OF THE INVENTION

In the first aspect thereof the invention relates to new palladium (I) complexes, provided with antitumour activity, having general formula (A) or (B).

The compounds of the invention are formed by heterocyclic units, in particular imidazolidines (general formula (A)) and benzimidazolidines (general formula (B)) with a carbon of the carbene type between two nitrogen atoms in the heterocyclic ring; the carbene carbon atoms complex two palladium atoms, which have a direct bond with each other, producing a dimeric species in which palladium is in oxidation state +1 (Pd(I)).

In general formulae (A) and (B), R1 is an alkyl radical or a phenyl radical optionally substituted with one or more C₁-C₃ alkyl groups. The preferred alkyl radicals for the purposes of the invention are tert-butyl and 1-adamantyl, whereas preferred aromatic radicals are 2,4,6-trimethylphenyl (commonly referred to as mesityl) and 2,6-diisopropylphenyl.

The substituents R2 and R3 may be a linear or branched C₁-C₆ alkyl radical. The subscript m, related to the substituents R2 in general formula (A), may be equal to 0, 1 or 2; the preferred case is m = 0, wherein the carbons of the imidazolidine ring are saturated with hydrogen atoms. Likewise, the subscript n, related to the substituents R3 in general formula (B), may be equal to 0, 1, 2, 3 or 4, and the preferred case is n = 0.

Finally, X is a pharmaceutically acceptable monovalent or bivalent anion, chosen among those known by the skilled in the art; preferred monovalent anions for the purposes of the invention are chloride, perchlorate (ClO₄⁻), tetrafluroborate (BF₄⁻) and hexafluorophosphate (PF₆⁻); examples of possible bivalent anions, typically used in the pharmaceutical sector, are sulphate, oxalate, maleate and fumarate.

The preferred compounds of the invention have the formulae A1 and B1 reported below: 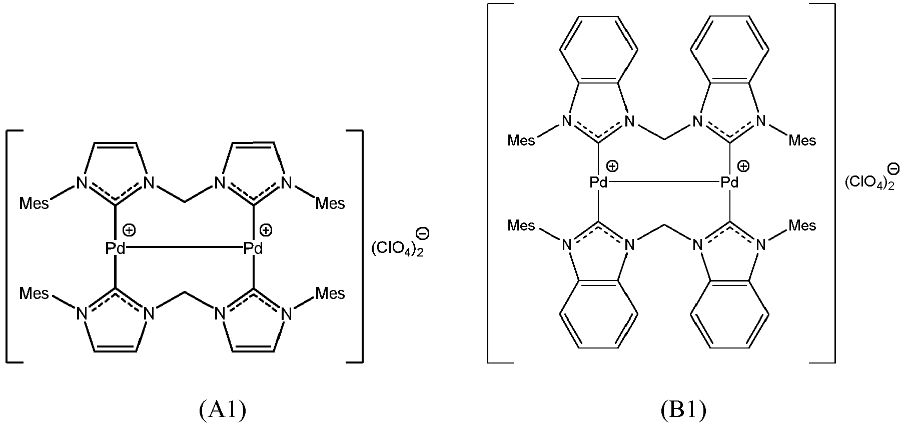 wherein Mes indicates the mesityl radical.

In its second aspect the invention relates to a process for the synthesis of the compounds of general formula (A) and (B), consisting in reacting a compound of general formula (C) or of general formula (D) with a palladium (II) complex, as reported above.

The compound of general formula (C) reported above, which is used as a reactant in the process of the invention for the preparation of a compound of general formula (A), can be obtained by reaction between the corresponding imidazolium salt and silver oxide according to the conditions reported in literature ("An unusually static, sterically hindered silver bis(n-heterocyclic carbene) complex and its use in transmetalation", Y. A. Wanniarachchi et al., Organometallics, 2004, 23, 5881-5884).

The compound of general formula (D) reported above, which is used as a reactant in the process of the invention for preparing a compound of general formula (B), can be synthesized, starting from the related bis-imidazolium salt, in quite similar conditions as those described for compound (C). For the preparation of the specific compounds (A1) and (B1), the compounds (C1) and (D1), the formula of which is shown below, are used as reactants:

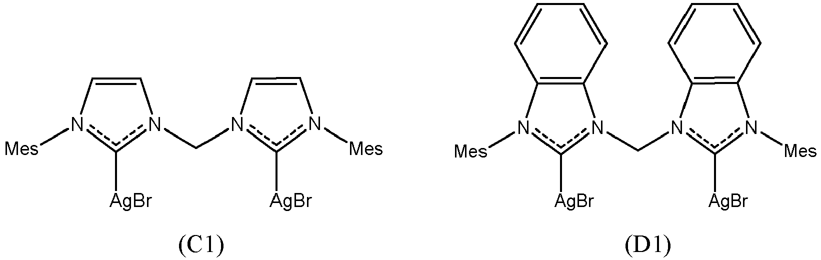

The second reactant of the process of the invention is a Pd(II) complex, indicated generally as Cpx(Pd(II)). The palladium complex must have one or more sufficiently labile coordination sites for the introduction of the biscarbene ligand and a sufficiently hindered allyl fragment.

Preferred palladium complexes are [Pd(µ-Cl)(η³-1,1-dimethylallyl)]₂ and [(MePyCH₂SPh)Pd(η³-1,1-dimethylallyl)]ClO₄, having the following structural formulae:

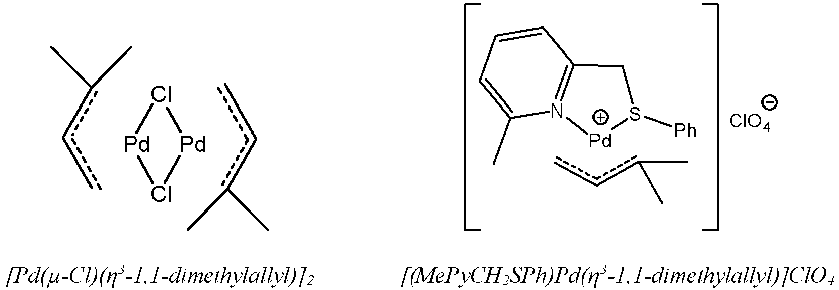

For simplicity, in the rest of the description, these two complexes shall be simply indicated as Pd(1,1-dimethylallyl)Clz and [Pd(NS)(1,1-dimethylallyl)]X.

These Pd(II) complexes can be prepared with methods known to those skilled in the art of coordination chemistry.

For example, the complex Pd(1,1-dimethylallyl)Clz can be prepared by suspending PdCl₂ powders in a solution of 1,1-dimethyl-propyl-1-ene in methanol; both these reactants are commonly available on the market.

Likewise, the complex [Pd(NS)(1,1-dimethylallyl)]X can be prepared by dissolving the complex Pd(1,1-dimethylallyl)Clz in dichloromethane in the presence of the ligand 2-(phenylthiomethyl)-6-methyl-pyridine, according to the methods described in the article "Mechanism of the reaction of allyl amination of Pd(II) allyl complexes containing chelating pyridine-chalcogen ligands. A surprisingly low influence of the chalcogen atom", L. Canovese, Polyhedron 20 (2001) 3171-3181. In particular, the synthesis of the ligand 2-(phenylthiomethyl)-6-methyl-pyridine consists of reacting thiophenol and 2-chloromethyl-6-methylpyridine hydrochloride in a solution of potassium hydroxide in dimethyl sulfoxide, followed by usual work-up operations (addition of water, separation of the phases, concentration of the organic phase and purification of the product by chromatography).

Finally, the reactant MX has the function of introducing the anion X into the reaction, to compensate for the charge of the Pd(I) complex that is produced in the process. As mentioned, the anion is selected from the pharmaceutically acceptable ones, and preferably from chloride, perchlorate, tetrafluoborate and hexafluorophosphate, whereas the cation is preferably selected from silver and alkali metals (Li, Na and K).

The reaction is carried out in an organic solvent, preferably dichloromethane, at a temperature between 0 °C and 35 °C, for times generally between 5 minutes and 1 hour. The stoichiometry of the reaction requires the use of the compound of formula (C) or (D) in a molar ratio 1:1 with respect to the Pd(II) complex when this contains a single Pd atom, and in a molar ratio 2:1 with respect to the Pd(II) complex when this contains two Pd atoms.

Finally, the invention relates to the Pd(I) complexes described above for use in the treatment of tumours by administration of said complexes to a patient affected by a tumour, possibly in combination with at least one cytostatic or cytotoxic agent and, possibly, in the form of a combined preparation for simultaneous, separate or sequential use in the antitumour therapy.

The complexes of the invention are particularly effective in the treatment of ovarian, cervical, colon and lung cancers.

Said complexes may be administered by the typical routes of chemotherapy, for example in the form of intravenous solutions (the most frequent), intramuscular or subcutaneous, or orally through capsules or tablets.

The invention will be further illustrated by the following examples.

### INSTRUMENTS, METHODS AND EXPERIMENTAL CONDITIONS ¹H-NMR and ¹³C-NMR:

Bruker 400 Avance spectrometer.

### XRD:

XRD1 beamline at Elettra Sincrotrone, Trieste (Italy).

### ANTIPROLIFERATIVE ACTIVITY:

Tecan analyser, mod. Infinite^{®} M1000.

### EXAMPLE 1

This example relates to the preparation of the compound (A1) of the invention.

In a 50 mL round-bottom flask, kept in an argon atmosphere, 0.0690 g (0.164 mmol) of the precursor Pd(1,1-dimethylallyl)Clz were dissolved in 15 mL of anhydrous dichloromethane; then 0.2485 g (0.3270 mmol) of compound (C1), previously dissolved in 5 mL of dichloromethane, and 0.0678 g (0.3270 mmol) of AgClO₄ were added thereto. The reaction mixture was kept under agitation for 15 minutes; during the reaction, the temperature fluctuated in the range 25 ± 2 °C. At the end of this period, the reaction mixture was filtered to remove the silver bromide that had formed. The volume of the solution obtained was reduced using a rotary evaporator. The addition of diethylether (Et₂O) induced the precipitation of a mixture of the compound (A1) (dimer complex of Pd(I)) and, as a by-product, of a palladium complex with a single metal centre.

The solid fraction was dissolved in a mixture of CH₂Cl₂/Et₂O (30 mL:70 mL) and the system was allowed to rest at 5 °C overnight. The selective precipitation of the dimeric species of Pd(I) (Compound (A1)) in the form of a pink solid was obtained, which was filtered with a Gooch crucible (yield: 0.0354 g). The compound is stable both in the solid state and in solution and was characterised using NMR spectroscopy.

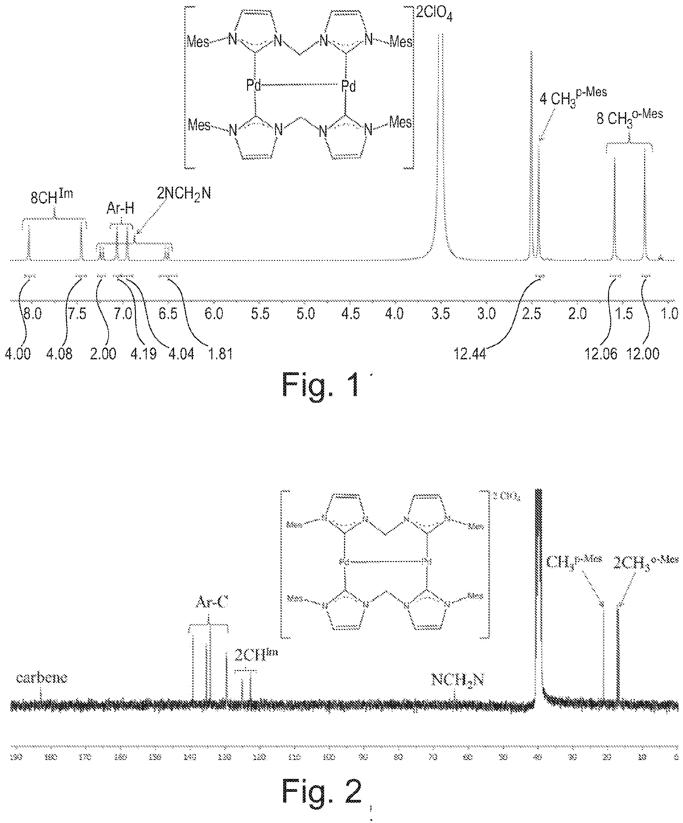

The ¹H-NMR and ¹³C{¹H}-NMR spectra of the compound are reported respectively in Figures 1 and 2. The listing and the attribution of the peaks in the two spectra are provided below.

¹H-NMR (300 MHz, d6-DMSO, T = 298 K, ppm) δ: 1.26 (s, 12H, 4 o-aryl-CH₃), 1.59 (s, 12H, 4 o-aryl-CH₃), 2.43 (s, 12H, 4 p-aryl-CH₃), 6.51 and 7.23 (AB system, J = 13.6 Hz, 4H, 2NCH₂N), 6.95-7.07 (8H, Ar-H), 7.45-8.03 (8H, CH^{Im}).

¹³C{¹H}-NMR (d6-DMSO, T = 298 K, ppm) δ: 16.8 (CH₃, o-Mesityl-CH₃), 17.2 (CH₃, o-Mesityl-CH₃), 21.2 (CH₃, p-Mesityl-CH₃), 64.0 (CH₂, NCH₂N), 122.6-125.1 (CH, CH=CH^{Im}), 129.6-139.3 (Ar-C), 182.8 (C, carbene).

### EXAMPLE 2

This example relates to an alternative preparation of the compound (A1) of the invention.

In a 50 mL round-bottom flask, kept in an argon atmosphere, 0.1603 g (0.3270 mmol) of the precursor [Pd(NS)(1,1-dimethylallyl)]Cl₄ were dissolved in 15 mL of dichloromethane anhydrous; then 0.2485 g (0.3270 mmol) of compound (C1), previously dissolved in 5 mL of dichloromethane, were added thereto. The reaction mixture was kept under agitation for 15 minutes; during the reaction, the temperature fluctuated in the range 25 ± 2 °C. At the end of this period, the reaction mixture was filtered to remove the formed silver bromide. The volume of the solution obtained was reduced using a rotary evaporator. The addition of diethylether induced the precipitation of a mixture of the compound (A1) (dimer complex of Pd(I)) and, as a by-product, of a palladium complex with a single metal centre.

The solid fraction was dissolved in a mixture of CH₂Cl₂/Et₂O (30 mL:70 mL) and the system was allowed to rest at 5 °C overnight. The selective precipitation of the dimeric species of Pd(I) (Compound (A1)) in the form of a pink solid was obtained, which was filtered with a Gooch crucible (yield: 0.0354 g). The compound is stable both in the solid state and in solution and was characterised using NMR spectroscopy. The ¹H-NMR and ¹³C{¹H}-NMR spectra of the compound obtained coincide with those of Example 1.

### EXAMPLE 3

This example relates to the structural characterization of the compound (A1) produced in Example 1.

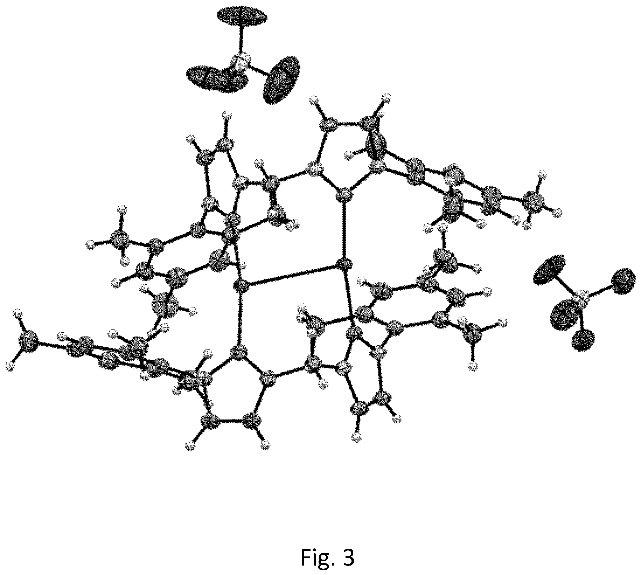

The crystallographic data relating to compound (A1) were collected at the temperature of 100 K at the XRD1 beamline of Elettra Sincrotrone of Trieste, using a monochromatic light with wavelength of 0.620 Å. Figure 3 shows a perspective view of the spatial structure of the compound obtained by X-ray analysis.

### EXAMPLE 4

This example relates to the evaluation of the therapeutic efficacy of the compounds of the invention.

Tests were performed for the purpose of determining the inhibitory concentration (IC₅₀) of a compound of the invention towards tumour cell lines and on a control cell line; for comparative purposes, the same tests were performed with the cisplatin compound.

The tested cell lines were: A2780, A2780cis, OVCAR5, OVCAR3 e KURAMOCHI (ovarian cancer), A549 (lung cancer), HeLa (cervical cancer), DLD1 (colon cancer) and MRC-5 (control; lung fibroblasts). The mother solutions were obtained in dimethyl sulfoxide for the Pd(I) compound and in H₂O for cisplatin.

The cells were kept, according to the protocols indicated by the suppliers, at 37 °C in an atmosphere containing 5% carbon dioxide. About 500 cells were plated on 96 different well-plates and kept under such conditions for 96 hours.

The viability of the cells was determined, after treatment of the cells with a 1:1 CellTiter-Glo^{®} solution (registered trademark of Promega Corp., Madison, Wisconsin, USA) and DPBS, with the Tecan Infinite^{®} M1000 instrument. The IC₅₀ values were calculated by the response curves to the dose. The means and standard deviations were obtained from triplicate measurements. The test results are shown in the following table.

**Table 1**

| **Cell line** | **IC₅₀** (***µM***) | |
|---|---|---|
| | **Cisplatin** | **A1** |
| A2780 | 1.0 ± 0.2 | 0.025± 0.007 |
| A2780cis | 43 ± 3 | 1.9 ± 0.2 |
| OVCAR5 | 5.2 ± 0.8 | 1.4 ± 0.3 |
| OVCAR3 | 2.2 ± 0.1 | 3.0 ± 0.2 |
| Kuramochi | 1.7 ± 0.3 | 0.38 ± 0.09 |
| HeLa | 11±2 | 3.5 ± 0.1 |
| A549 | 6±3 | 0.38±0.02 |
| DLD1 | 4.2 ± 0.5 | 2.8 ± 0.6 |
| MRC-5 | 14 ± 1 | >100 |

The values reported in table 1 show that the compound of the invention is very active towards all the examined tumour lines. Furthermore, it was substantially inactive (IC₅₀ >100 µM) towards normal cells (MRC-5 lung fibroblasts), and therefore does not have the toxicity side effects encountered with cisplatin.

## Claims

1. Compound of general formula (A) or of general formula (B): 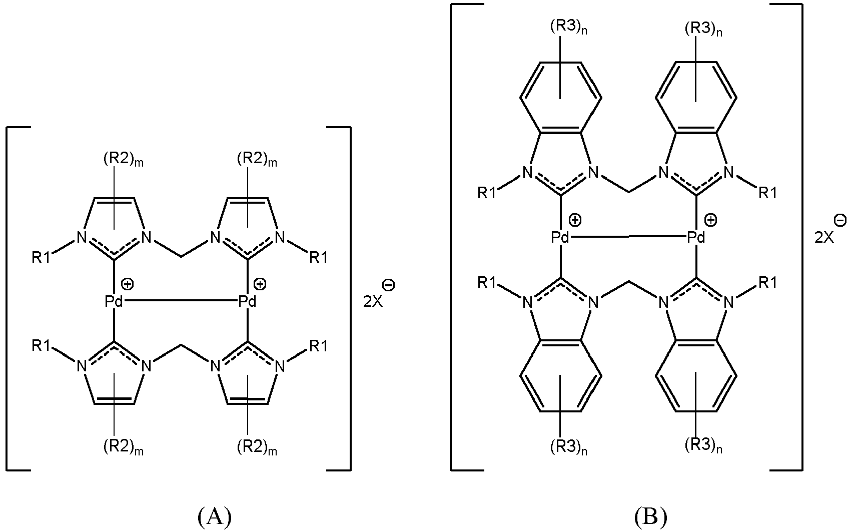 wherein:
- R1 is an alkyl radical or a phenyl radical optionally substituted with one or more C₁-C₃ alkyl groups;
- R2 is a linear or branched C₁-C₆ alkyl radical;
- R3 is a linear or branched C₁-C₆ alkyl radical;
- m = 0, 1 or 2;
- n = 0, 1, 2, 3 or 4;
- 2X^{Θ} indicates two pharmaceutically acceptable monovalent anions or a pharmaceutically acceptable bivalent anion.

2. Compound according to claim 1, wherein R1 is selected from tert-butyl, 1-adamantyl, 2,4,6-trimethylphenyl (mesityl) and 2,6-diisopropylphenyl.

3. Compound according to any one of claims 1 or 2, wherein said pharmaceutically acceptable monovalent anion is selected from chloride (Cl⁻), perchlorate (ClO₄⁻), tetrafluoborate (BF₄⁻) and hexafluorophosphate (PF₆⁻).

4. Compound according to claim 1, wherein R1 is 2,4,6-trimethylphenyl (mesityl), m = 0, n = 0 and the monovalent anion is perchlorate.

5. Process for the synthesis of a compound of any one of claims 1 to 4, consisting in reacting a compound of general formula (C) or a compound of general formula (D) with a palladium (II) complex, according to one of the following diagrams: 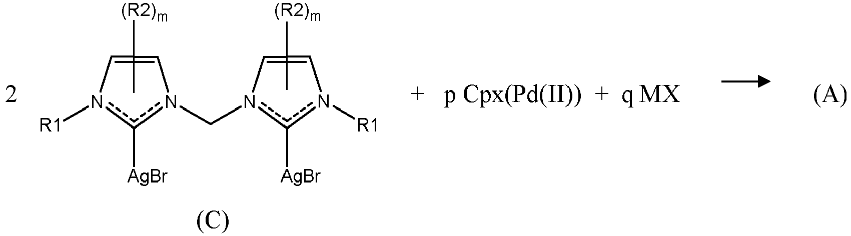 or 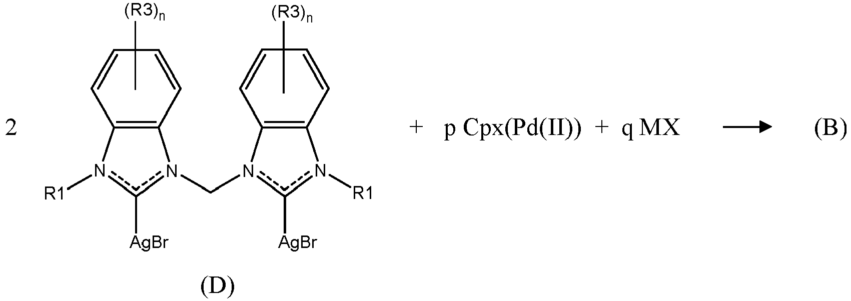 wherein:
- R1 is an alkyl radical or a phenyl radical optionally substituted with one or more C₁-C₃ alkyl groups;
- R2 is a linear or branched C₁-C₆ alkyl radical;
- R3 is a linear or branched C₁-C₆ alkyl radical;
- m = 0, 1 or 2;
- n = 0, 1, 2, 3 or 4;
- Cpx(Pd(II)) is a palladium (II) complex;
- MX is a salt of a pharmaceutically acceptable monovalent or bivalent anion;
- p = 2 if Cpx(Pd(II)) contains a single Pd atom, and p = 1 if Cpx(Pd(II)) contains two Pd atoms;
- q = 1 when said anion is a bivalent anion, and q = 2 when said anion is a monovalent anion.

6. Process according to claim 5, wherein the compound of formula (C1) is used as compound of general formula (C), or the compound of formula (D1) is used as compound of general formula (D):

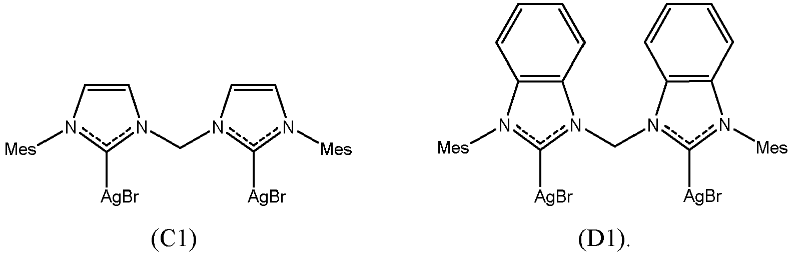

7. Process according to any one of claims 5 or 6, wherein the palladium (II) complex used as a reactant is selected from [Pd(µ-Cl)(η³-1,1-dimethylallyl)]₂ and [(MePyCH₂SPh)Pd(η³-1,1-dimethylallyl)]ClO₄:

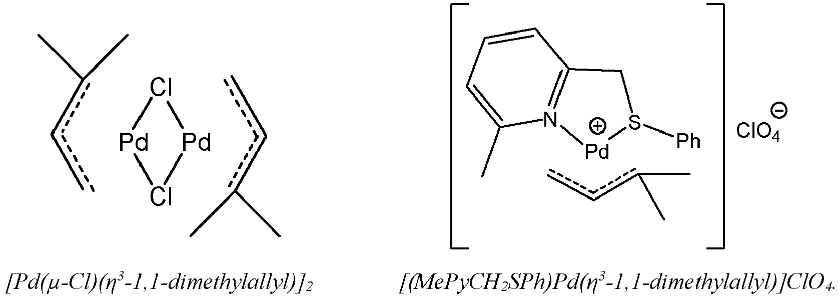

8. Process according to any one of claims 5 to 7, wherein the cation of the MX salt is selected from silver, lithium, sodium and potassium.

9. Process according to any one of claims 5 to 8, wherein the reaction of a compound of general formula (C) or of general formula (D) with a palladium (II) complex is carried out in an organic solvent, preferably dichloromethane, at a temperature between 0 °C and 35 °C for a time between 5 minutes and an hour.

10. Compound of general formula (A) or (B) as defined in claim 1 for use as an antitumour therapeutic agent.

11. Compound according to claim 10 for use in the treatment of ovarian, cervical, colon and lung cancer.

## Patentansprüche

1. Verbindung der allgemeinen Formel (A) oder der allgemeinen Formel (B): 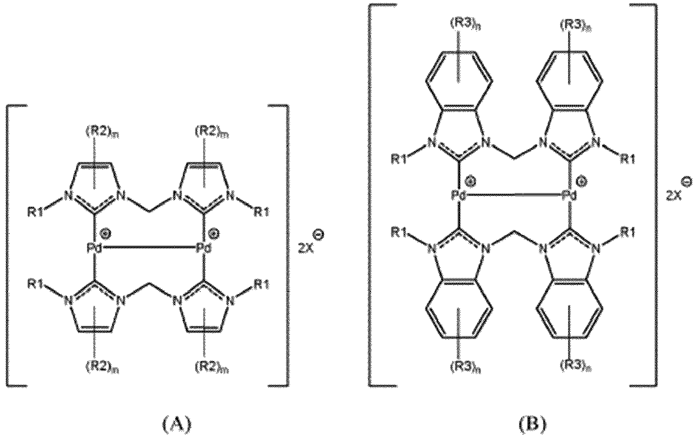 wobei:
- R1 ein Alkylrest oder ein Phenylrest ist, der gegebenenfalls mit einer oder mehreren C₁-C₃ -Alkylgruppen substituiert ist;
- R2 ein linearer oder verzweigter C₁-C₆ -Alkylrest ist;
- R3 ein linearer oder verzweigter C₁-C₆ -Alkylrest ist;
- m = 0, 1 oder 2 ist;
- n = 0, 1, 2, 3 oder 4 ist;
- 2X^{Θ} zwei pharmazeutisch annehmbare einwertige Anionen oder ein pharmazeutisch annehmbares zweiwertiges Anion bezeichnet.

2. Verbindung nach Anspruch 1, wobei R1 ausgewählt ist aus tert-Butyl, 1-Adamantyl, 2,4,6-Trimethylphenyl (Mesityl) und 2,6-Diisopropylphenyl.

3. Verbindung nach einem der Ansprüche 1 oder 2, wobei das pharmazeutisch annehmbare einwertige Anion ausgewählt ist aus Chlorid (Cl⁻), Perchlorat (ClO₄⁻), Tetrafluorborat (BF₄⁻) und Hexafluorphosphat (PF₆⁻).

4. Verbindung nach Anspruch 1, worin R1 2,4,6-Trimethylphenyl (Mesityl) ist, m = 0, n = 0 und das einwertige Anion Perchlorat ist.

5. Verfahren zur Synthese einer Verbindung nach einem der Ansprüche 1 bis 4, das darin besteht, eine Verbindung der allgemeinen Formel (C) oder eine Verbindung der allgemeinen Formel (D) mit einem Palladium(II)-Komplex nach einem der folgenden Schemata umzusetzen: 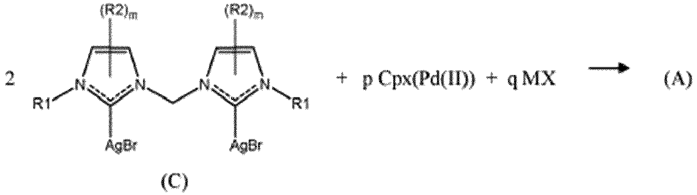 oder 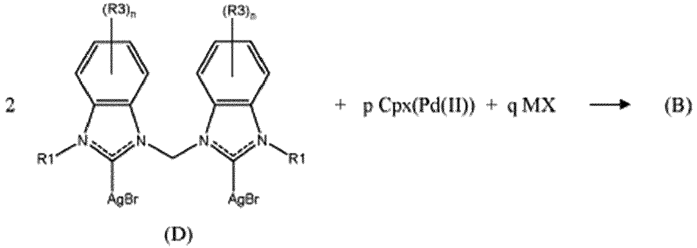 wobei:
- R1 ein Alkylrest oder ein Phenylrest ist, der gegebenenfalls mit einer oder mehreren C₁-C₃ -Alkylgruppen substituiert ist;
- R2 ein linearer oder verzweigter C₁-C₆ -Alkylrest ist;
- R3 ein linearer oder verzweigter C₁-C₆ -Alkylrest ist;
- m = 0, 1 oder 2 ist;
- n = 0, 1, 2, 3 oder 4 ist;
- Cpx(Pd(II)) ein Palladium(II)-Komplex ist;
- MX ein Salz eines pharmazeutisch annehmbaren einwertigen oder zweiwertigen Anions ist;
- p = 2 ist, wenn Cpx(Pd(II)) ein einzelnes Pd-Atom enthält, und p = 1 ist, wenn Cpx(Pd(II)) zwei Pd-Atome enthält;
- q = 1 ist, wenn das Anion ein zweiwertiges Anion ist, und q = 2 ist, wenn das Anion ein einwertiges Anion ist.

6. Verfahren nach Anspruch 5, wobei die Verbindung der Formel (C1) als Verbindung der allgemeinen Formel (C) verwendet wird oder die Verbindung der Formel (D1) als Verbindung der allgemeinen Formel (D) verwendet wird:

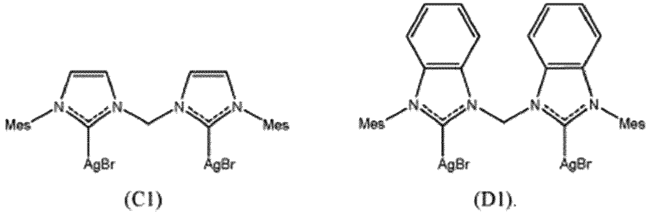

7. Verfahren nach einem der Ansprüche 5 oder 6, wobei der als Reaktant verwendete Palladium(II)-Komplex ausgewählt ist aus [Pd(µ-Cl)(η³-1,1-dimethylallyl)]₂ und [(MePyCH₂SPh)Pd(η³-1,1-dimethylallyl)]ClO₄:

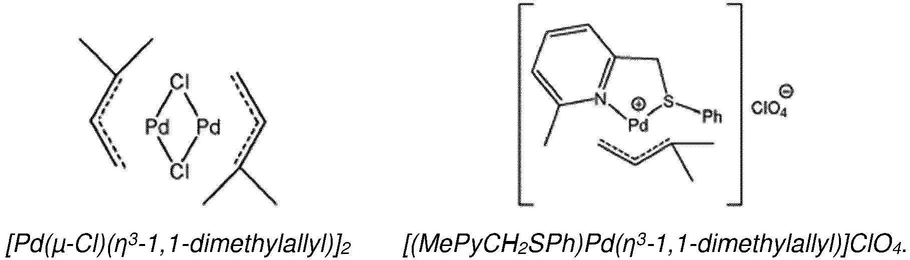

8. Verfahren nach einem der Ansprüche 5 bis 7, wobei das Kation des MX-Salzes ausgewählt ist aus Silber, Lithium, Natrium und Kalium.

9. Verfahren nach einem der Ansprüche 5 bis 8, **dadurch gekennzeichnet, dass** die Reaktion einer Verbindung der allgemeinen Formel (C) oder der allgemeinen Formel (D) mit einem Palladium(II)-Komplex in einem organischen Lösungsmittel, vorzugsweise Dichlormethan, bei einer Temperatur zwischen 0 °C und 35 °C für eine Zeit zwischen 5 Minuten und einer Stunde durchgeführt wird.

10. Verbindung der allgemeinen Formel (A) oder (B), wie in Anspruch 1 definiert, zur Verwendung als therapeutisches Antitumormittel.

11. Verbindung nach Anspruch 10 zur Verwendung bei der Behandlung von Eierstock-, Gebärmutterhals-, Kolon- und Lungenkrebs.

## Revendications

1. Composé de formule générale (A) ou de formule générale (B) : 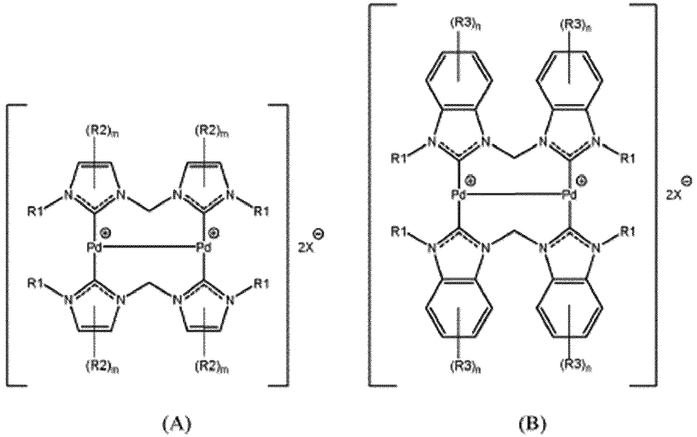 dans lequel :
- R1 est un radical alkyle ou un radical phényle éventuellement substitué par un ou plusieurs groupes alkyle en C₁-C₃ ;
- R2 est un radical alkyle linéaire ou ramifié en C₁-C₆ ;
- R3 est un radical alkyle linéaire ou ramifié en C₁-C₆ ;
- m = 0, 1 ou 2 ;
- n = 0, 1, 2, 3 ou 4 ;
- 2X^{Θ} indique deux anions monovalents pharmaceutiquement acceptables ou un anion bivalent pharmaceutiquement acceptable.

2. Composé selon la revendication 1, dans lequel R1 est choisi parmi le tert-butyle, le 1-adamantyle, le 2,4,6-triméthylphényle (mésityle) et le 2,6-diisopropylphényle.

3. Composé selon l'une quelconque des revendications 1 ou 2, dans lequel ledit anion monovalent pharmaceutiquement acceptable est choisi parmi le chlorure (Cl⁻), le perchlorate (ClO₄⁻), le tétrafluoborate (BF₄⁻) et l'hexafluorophosphate (PF₆⁻).

4. Composé selon la revendication 1, dans lequel R1 est le 2,4,6-triméthylphényle (mésityle), m = 0, n = 0 et l'anion monovalent est le perchlorate.

5. Procédé pour la synthèse d'un composé de l'une quelconque des revendications 1 à 4, consistant à faire réagir un composé de formule générale (C) ou un composé de formule générale (D) avec un complexe de palladium (II), selon l'un des schémas suivants : 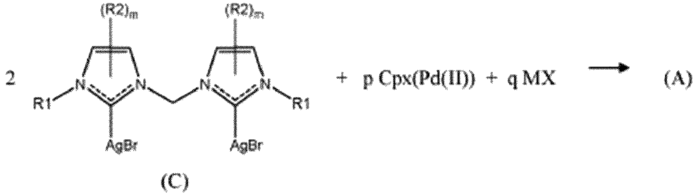 ou 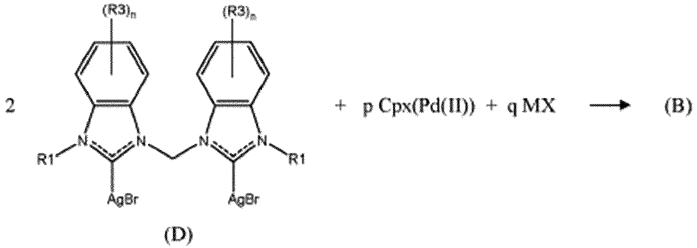 dans lequel :
- R1 est un radical alkyle ou un radical phényle éventuellement substitué par un ou plusieurs groupes alkyle en C₁-C₃ ;
- R2 est un radical alkyle linéaire ou ramifié en C₁-C₆ ;
- R3 est un radical alkyle linéaire ou ramifié en C₁-C₆ ;
- m = 0, 1 ou 2 ;
- n = 0, 1, 2, 3 ou 4 ;
- Cpx(Pd(II)) est un complexe de palladium (II) ;
- MX est un sel d'un anion monovalent ou bivalent pharmaceutiquement acceptable ;
- p = 2 si Cpx(Pd(II)) contient un seul atome de Pd, et p = 1 si Cpx(Pd(II)) contient deux atomes de Pd ;
- q = 1 lorsque ledit anion est un anion bivalent, et q = 2 lorsque ledit anion est un anion monovalent.

6. Procédé selon la revendication 5, dans lequel le composé de formule (C1) est utilisé comme composé de formule générale (C), ou le composé de formule (D1) est utilisé comme composé de formule générale (D) :

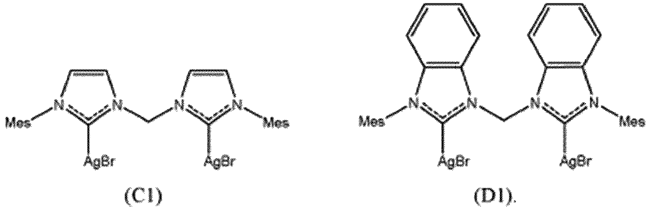

7. Procédé selon l'une quelconque des revendications 5 ou 6, dans lequel le complexe de palladium (II) utilisé comme réactif est choisi parmi [Pd(µ-Cl)(η³-1,1-diméthylallyl)]₂ et [(MePyCH₂SPh)Pd(η³-1,1-diméthylallyl)]ClO₄ :

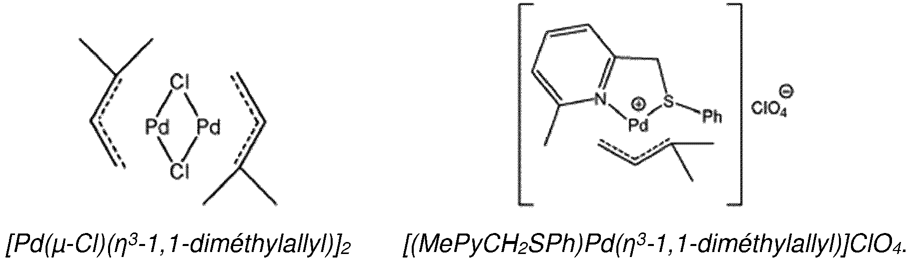

8. Procédé selon l'une quelconque des revendications 5 à 7, dans lequel le cation du sel MX est choisi parmi l'argent, le lithium, le sodium et le potassium.

9. Procédé selon l'une quelconque des revendications 5 à 8, dans lequel la réaction d'un composé de formule générale (C) ou de formule générale (D) avec un complexe de palladium (II) est effectuée dans un solvant organique, de préférence le dichlorométhane, à une température comprise entre 0 °C et 35 °C pendant une durée comprise entre 5 minutes et une heure.

10. Composé de formule générale (A) ou (B) tel que défini dans la revendication 1 pour une utilisation comme agent thérapeutique antitumoral.

11. Composé selon la revendication 10 pour une utilisation dans le traitement du cancer de l'ovaire, du col de l'utérus, du colon et du poumon.
